# EUROPEAN PATENT APPLICATION

(11) **EP 1 659 512 A2**
(43) Date of publication of application: **24.05.2006**
(21) Application number: 05111113.6
(22) Date of filing: 22.11.2005
(51) Int. Cl.: G06F 19/00

(54) **Emergency station kiosk and related methods**

(30) Priority: 22.11.2004 US 996296
(71) Applicant: Rao, John, Tempe AZ 85283 (US)
(72) Inventor: Rao, John, Tempe AZ 85283 (US)
(74) Representative: Curley, Donnacha John

(57) **Abstract**

Particular implementations are particularly useful in providing a system that allows the secure distribution of medical documents through a medical station. In some implementations, the medical station includes a user interface and a camera that captures an image and attaches the image to a medical document. In order to receive a medical document through the system, a user requests a medical examination, enters information into the system by answering questions, and by using medical implements, and then requests the medical document. A picture of the customer or other patient is captured for attachment to the medical document. Another implementation allows a station user to communicate directly with a medical professional during the examination process through a medical interface on the station. Some particular implementations arc configured as mobile medical stations.

## Description

This application is related to another patent application by Rao entitled "PRESCRIPTION DRUG DISTRIBUTION SYSTEM AND METHODS," serial number 10/389,363, filed on March 14, 2003, the disclosure of which are hereby incorporated herein by reference.

### BACKGROUND

### Technical Field

Aspects of this disclosure relate generally to medical stations and kiosks and to related methods for distributing medical documents. Aspects relate more specifically to implementations of a medical kiosk through which medical services and medical documents are provided, and to implementations of a mobile emergency medical station.

### Background Art

With the advent of the Internet and other new technologies, it has become possible to make medical services available where they have not been available in the past. This has been illustrated partly by the growing industry of mail order and online pharmacies. These companies allow customers to research, select and request a desired drug. The customer then answers questions posed by an online doctor and the prescription for the requested drug is granted. These companies, however, can only grant prescriptions to a group of prescription drugs known in the industry as "lifestyle" drugs that are not necessary for the health of the patient, but may improve the quality of life for the patient. Some examples of lifestyle drugs include, but are not limited to, drugs for weight loss, hair loss, erectile dysfunction, pain relief from arthritis, allergies, herpes, birth control, skin care, smoking, and the like. Though a step in the right direction, these companies do not provide access to medical care which is necessary for the health of the patient.

US Patents No. 6,046,761 and 5,801,755 awarded to Echerer detail an interactive communication system for providing medical services to remotely located patients. This system provides video and audio interaction between the patient and a medical practitioner who may be many miles away.

A problem, however, with both the internet pharmacies and the Echerer system is that they only confirm that the customer has the right ID card, which could easily be faked. Accordingly, what is needed is a system that allows for the convenience of internet pharmacies and the Echerer system, yet provides security so that the customer obtaining the medical service is permanently associated with the result of the service regardless of the ID card shown.

Wireless communication technologies such as cellular telephones, satellites, WiFi, WiMax, Bluetooth, and the like have significantly spread in availability and improved in connectivity over the past year. Despite this rapid development of communication technologies, the availability of medical diagnosis and treatment to remote areas, however, and particularly in emergency situations, continues to be a problem for many.

### SUMMARY

Some aspects of this disclosure relate to methods and systems for distributing medical documents and/or medical services through a medical station or kiosk. Aspects of the disclosure include a station that contains a user interface. Other aspects of the disclosure relate to a mobile medical station.

In particular implementations, the interface allows station users to communicate with medical professionals to obtain medical documents. Examples of medical documents include prescriptions, letters concerning the health of the customer, referrals, test requests and the like. Specific implementations are particularly useful for providing medical services conveniently and at remote locations. In particular implementations, a photograph is taken of the customer requesting the medical document and that photograph is then attached to the medical document so that the identity of the customer to which the medical document corresponds can be confirmed.

Station users may approach or enter a medical station and access the medical interface. The medical interface may consist of audio and visual capabilities such as a monitor which allows the station user to see and hear a medical professional. It may also contain a video camera which allows a remote medical professional to see and hear the station user or someone near the station user. The medical interface may also have medical implements, such as a stethoscope, attached or attachable. This allows the station user to conduct a medical examination of him/herself or someone near the station user while being supervised by the medical professional. The data from the examination is then transmitted to the medical professional or otherwise stored in a local or remote in order to allow the medical professional to determine the health and/or condition of the patient. The medical professional may then issue a medical document to the station user if appropriate.

In particular implementations, as the medical document is requested, such as at a point during the examination, a photograph may be taken of the station user or other patient. This photograph may be associated with the medical document in order to provide anyone presented with the document a way to determine whether or not the person presenting the document is the patient who was actually examined, or to identify a particular condition of the patient or a portion of the patient. This helps to prevent fraud and mistreatment of medical patients, and to obtain fleeting condition information relating to the patient.

In particular implementations, the medical station is formed as a mobile kit with wireless communication capabilities to permit the medical station to be used for remote locations and for emergency situations where a medical professional is needed but not immediately available. Mobile implementations may include a collection of medical implementations conveniently stored within a carrying case and selectively connectable to a station terminal with remote communication capabilities. Particular implementations of the mobile medical station kit include, among others, uses for automobile, airplane, retail store, and traveling doctor implementations.

The invention also provides for a mobile emergency medical kit/response system and a method using same in accordance with the following statements of invention:
1. A mobile emergency medical kit comprising:
   a plurality of electronic medical implements each configured to receive external stimuli and transmit electronic data indicative of the external stimuli, the plurality of electronic medical implements including at least two of a stethoscope, an otoscope, a thermometer, a camera, a blood pressure monitor, a pulse monitor, and a blood composition monitor;
   an electronic data collector comprising a data port, the collector configured to:
      a) selectively receive the data transmitted from each of the plurality of electronic medical implements and transmit electronic medical data from the electronic data collector with a unique identification code via a communication signal to an emergency data system for review by a medical professional, wherein the data is transmitted wirelessly for at least 2 kilometers from the collector; and
      b) receive communications from the medical professional relating to the data transmitted; and
   a storage container sized to store at least the plurality of electronic medical implements.
2. The mobile emergency medical kit of statement 1, wherein the data is transmitted wirelessly for at least 10 kilometers from the collector.
3. The mobile emergency medical kit of statement 1, wherein the data is transmitted wirelessly for at least 25 kilometers from the collector.
4. The mobile emergency medical kit of statement 1, wherein the data is transmitted wirelessly for at least 40 kilometers from the collector.
5. The mobile emergency medical kit of statement 1, wherein the electronic data collector further comprises an emergency button configured to transmit an emergency signal to the emergency data system and initiate accelerated access to the medical professional.
6. The mobile emergency medical kit of statement 1, wherein the plurality of medical implements further comprises at least one of an identification card reader, and a directional light.
7. The mobile emergency medical kit of statement 1, wherein the electronic data collector is a handheld electronic data collector and the storage container is further sized to store the handheld electronic data collector.
8. The mobile emergency medical kit of statement 1, wherein the electronic data collector is at least partially built into a vehicle.
9. The mobile emergency medical kit of statement 8, wherein the vehicle is an automobile or an airplane.
10. The mobile emergency medical kit of statement 1, wherein at least one of the electronic medical implements includes an electronic data plug and the electronic data collector comprises at least one electronic data port configured to receive the electronic data plug.
11. The mobile emergency medical kit of statement 1, wherein at least one of the electronic medical implements includes wireless communication capability and the electronic data collector is configured to receive wireless communication from the electronic medical implement.
12. A mobile emergency medical response system comprising:
   a plurality of mobile emergency medical kits, each kit having an electronic data collector with a capacity to transmit wireless communications and to receive wireless communications, the transmitted wireless communications including an identifier specifically identifying a particular kit from which the wireless communication originated, and a plurality of electronic medical implements configured to receive external stimuli and transmit electronic data indicative of the external stimuli;
   an emergency data system configured to receive the wireless communications from the electronic data collectors, uniquely identify a source of the wireless communication, interpret the received communications, and display an interpretation of the data on at least one of a plurality of medical professional terminals associated with the emergency data system, each medical professional terminal configured to display the data interpretation and to receive a communication from a medical professional associated with the medical professional terminal and relay the communication to the source.
13. The mobile emergency medical response system of statement 12, wherein the plurality of electronic medical implements includes at least two of a stethoscope, an otoscope, a thermometer, a camera, a blood pressure monitor, a pulse monitor, and a blood composition monitor.
14. The mobile emergency medical response system of statement 12, wherein each kit includes an electronic data collector with a capacity to transmit wireless communications for at least 10 kilometers.
15. The mobile emergency medical response system of statement 12, wherein the electronic data collector is a handheld electronic data collector.
16. The mobile emergency medical response system of statement 12, wherein the electronic data collector is at least partially built into a vehicle.
17. The mobile emergency medical response system of statement 16, wherein the vehicle is an automobile or an airplane.
18. The mobile emergency medical response system of statement 12, wherein at least one of the electronic medical implements includes an electronic data plug and the electronic data collector comprises at least one electronic data port configured to receive the electronic data plug.
19. The mobile emergency medical response system of statement 12, wherein at least one of the electronic medical implements includes wireless communication capability and the electronic data collector is configured to receive wireless communication from the electronic medical implement.
20. A method of using a mobile emergency medical kit, the method comprising:
   accessing an electronic data collector included in the mobile emergency medical kit, the electronic data collector configured for wireless communication to a remote emergency data system;
   contacting a medical professional at the remote emergency data system through the electronic data collector;
   associating a medical implement with the electronic data collector;
   collecting medical data through the medical implement; and
   transmitting the collected medical data to the medical professional through the electronic data collector through wireless communication.

The aspects, implementations and examples set forth in this disclosure were presented in order to explain some practical applications of a medical kiosk to thereby enable those of ordinary skill in the art to make and use a medical kiosk having the advantages disclosed herein. However, those of ordinary skill in the art will recognize that the foregoing description and examples have been presented for the purposes of illustration and example only. The description as set forth is not intended to be exhaustive or to limit the invention to the precise form disclosed. Many modifications and variations are possible in light of the teachings above without departing from the spirit and scope of the forthcoming claims. For example, it should be clear to those of ordinary skill in the art from the disclosure that many aspects of the system, medical documents and mobile medical kit may be used independently to increase security, prevent fraud and expand the ability of medical professionals to treat patients.

These general and specific aspects may be implemented using a system, a method, and/or a computer program and electronic equipment, or any combination of systems, methods, and/or computer programs and electronic equipment. Additionally, the foregoing and other aspects, features, and advantages will be apparent from the DESCRIPTION and DRAWINGS, and from the CLAIMS.

### BRIEF DESCRIPTION OF THE DRAWINGS

Implementations will hereinafter be described in conjunction with the appended DRAWINGS, where like designations denote like elements, and:
FIG. 1 is a perspective view of an implementation of a medical station;
FIG. 2 is a side view of an implementation of a medical station configured as a booth;
FIG. 3 is a flow chart of an implementation of medical document distribution method;
FIG. 4 is an implementation of a prescription generated as a medical document;
FIG. 5 is a block diagram of a first implementation of a medical document distribution system;
FIG. 6 is a block diagram of a second implementation of a medical document distribution system;
FIGs. 7a, 7b and 7c are, respectively, representative illustrations of a mobile medical kit in open, rolled side, and rolled end views;
FIG. 8 is an illustration of an implementation of a mobile diagnostic tool processor;
FIG. 9 is an illustration of a dash board of a vehicle comprising a built-in mobile diagnostic tool processor;
FIG. 10 is a block diagram of an implementation of a mobile medical station; and
FIG. 11 is a flow chart of an implementation of use of a mobile emergency medical station.

### DESCRIPTION

As discussed above, implementations of particular aspects in this disclosure may relate to methods for distributing medical documents and services through the use of a medical kiosk. Other implementations may relate to mobile medical stations. Some aspects relate to all implementations and others relate to only some.

Examples of medical documents include prescriptions, letters concerning the health of the customer, medical referrals, test requests and the like. Through the use of the aspects included within implementations described herein, convenience, accessibility and security in providing medical services are increased. Throughout this description, "kiosk" and "station" may be used broadly and interchangeably to refer to any location through which information may be provided or received. With the implementation of the newest forms of communication presently available, broadband streaming data service with sufficient bandwidth now enables reliable transfer of significant amounts of data over wireless or partially wireless networks where previously such transfer was not possible or unreliable. Accordingly, those of ordinary skill in the data communications art will readily understand without further explanation that the systems and implementations described herein are examples of both wired and wireless systems and that the examples of wired systems may be implemented as wireless systems.

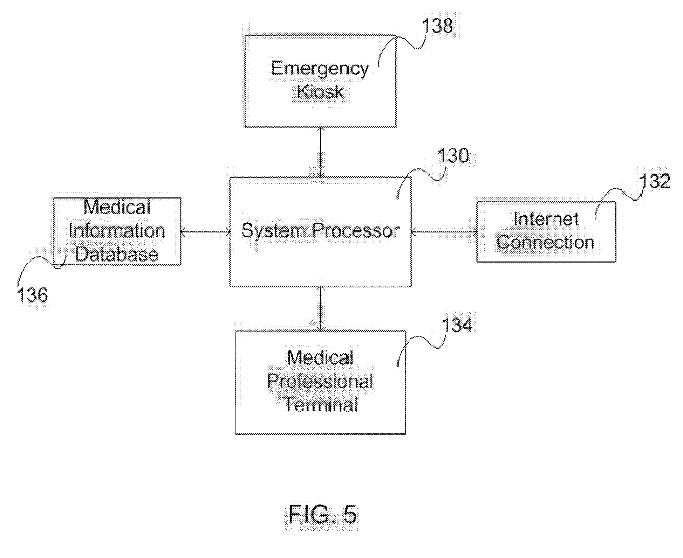

FIG. 5 illustrates a first implementation of a system for distributing prescription medications and medical services through the use of a medical kiosk 138. The medical kiosk 138 communicates with a system processor 130 which also communicates with a medical professional terminal 134. This allows a medical professional to communicate with a customer at a kiosk 138. The kiosk 138 allows a medical professional to conduct a remote medical examination and to issue medical documents from a remote location. The system may also include access to the internet 132, or other information network, and to a medical information database 136. Communications with a medical professional may be accomplished through a medical kiosk 138, whether stationary or mobile.

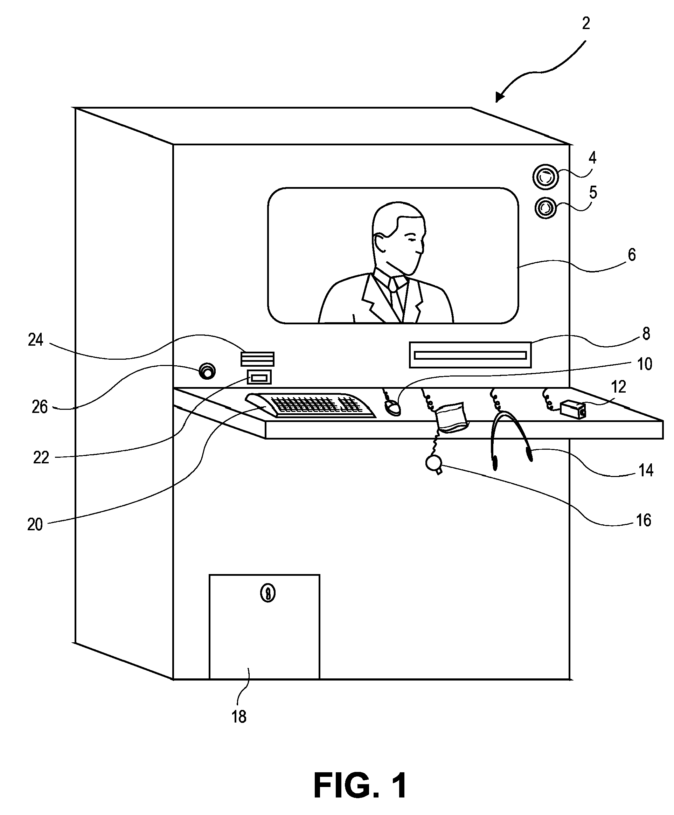

FIG. 1 illustrates a particular example of a medical kiosk 2. Kiosks having a fixed connection to a computer network or for connection to the Internet are known in the art and are available for purchase through kiosk companies such as Affordable Kiosks on the Internet at affordablekiosks.com. Adapting a system to enable wireless access to a computer network or for connection to the Internet is known to those of ordinary skill in the art familiar with recent wireless communication technologies such as WiFi and WiMax. Conventional kiosks include an input device such as a computer keyboard 20, which may also include a cursor controller 10 such as a mouse, touch-pad or track ball. The kiosk 2 of the present invention also includes a display 6, a credit card reader 24 and/or a currency acceptor 22, and a camera 4. The kiosk 2 may also contain an emergency button 26, a portable video camera 12, an emergency first aid kit 18 and other portable medical implements such as a stethoscope 14, blood pressure cuff 16, otoscope, ophthalmoscope, tuning fork and the like. An x-ray machine and a MRI machine may also be attached to the kiosk 2 for rapid data transfer to the doctor.

The display 6, credit card reader 24, currency acceptor 22, emergency button 26, medical implements 14 and 16, portable video camera 12, camera 4 and the like, are all parts of a customer interface. The customer interface consists of any devices through which the customer interacts with the kiosk, a computer or the distribution system.

The display 6 may be of a kind typical to kiosks for viewing images transmitted to the display 6. Additionally, as is common with kiosks, the display 6 may be a touch sensitive screen for interacting with the kiosk 2. In particular implementations using a touch screen, it may not be necessary to include a keyboard because simple interaction may be accomplished through the touch screen. In other implementations, however, both a touch sensitive display 6 and a keyboard 20 and/or a cursor controller 10, will be used in combination to enable interaction. In some other more simple implementations, a simple 12 to 20 key keypad may be all that is needed. Some form of verbal interaction or sound receiving and/or transmitting device (e.g. a speaker or microphone on the kiosk or a telephone handset), may be used to enable a customer to verbally interact with a person or system associated with the kiosk 2. It will be understood to those of ordinary skill in the art that instructions may be given and questions asked to the customer through the display 6, a verbal interaction device, a touch screen display 6 or a keyboard 20. Numerous combinations of interaction using these interaction tools may also be used to accomplish various purposes and achieve desired advantages. A verbal interaction device, however, gives the particular ability to enable easy interaction with the system or a live person, such as a pharmacist, doctor, medical professional or help desk, to ask questions, or for the doctor or medical professional to ask questions and examine the customer.

The camera 4 may be included in the kiosk 2 of implementations to capture a facial image of the customer requesting the medical document proximate the time the customer requests the document. Capturing a facial image of the customer who requested the medical document and of whom the medical examination was taken may be accomplished in a number of ways known to those of ordinary skill in the art and may include, without limitation stationary or moving digital or analog photography as well as other forms of capturing an image of the customer. As used herein, capturing an image of the customer "proximate" the time the customer requests the medical document means capturing the image of the customer at or around the time the customer requests the document, is examined, and/or reviews the information at the terminal, and does not include capturing an image of the customer which was taken at a previous time, such as a drivers license photo, or merely transferring a previously captured image of the customer on file. The picture feature of the invention is intended to provide added security and, therefore, should be an image of the actual customer of the medical document.

A video camera 5 may also be included in the kiosk 2 in particular implementations. The video camera 5 allows the customer to be viewed by a medical professional such as a doctor, emergency medical technician or nurse practitioner, among others. The video camera 5 may also be moveable and zoomable. It allows the customer and the medical professional to interact, the customer showing the medical professional various symptoms and the medical professional asking questions and giving directions to the customer. The video camera 5 would also allow the medical professional to supervise the customer's use of any medical implements attached to the kiosk 2 in order to assure that the customer takes the readings appropriately and gathers the correct information. The video camera 5 and other electronically recorded implement data would also provide a record of the examination that may be stored electronically and recalled later. The camera 4 and the video camera 5 may be combined into a single camera that takes videos but is also capable of capturing still images.

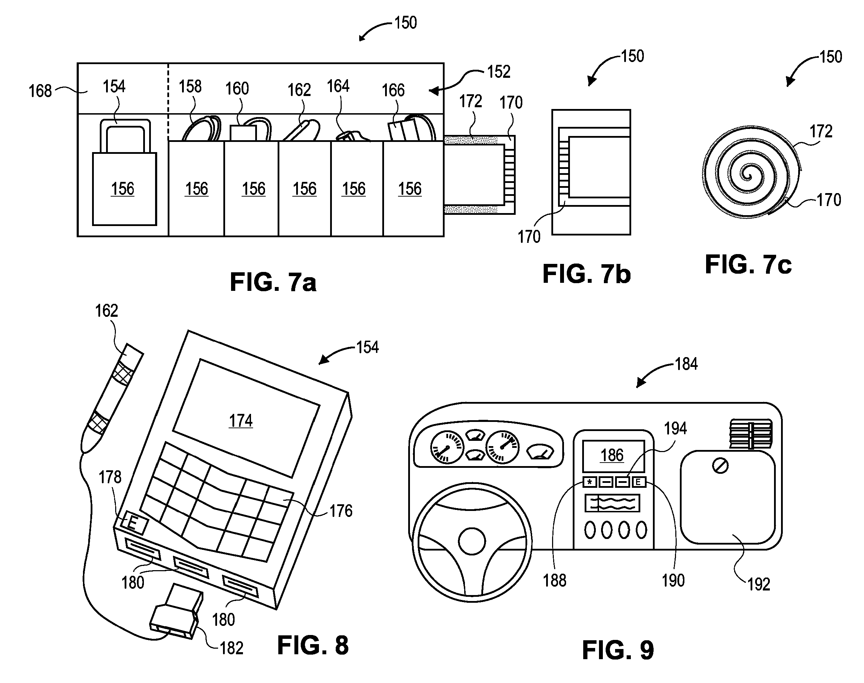

The kiosk 2 may also include an emergency button 26. The emergency button 26 signals a remote medical professional that a medical emergency is underway. Medical professionals then immediately report to a remote terminal which allows them to interact with the customer. Along with an emergency button 26, the kiosk 2 may contain an emergency first aid kit 18. This first aid kit 18 may be stored in a compartment in the kiosk 2. The remote medical professional would then be able to direct the treatment of the customer undergoing the medical emergency. While any of the implementations described herein may be adapted for use in emergency situations, the implementations described with reference to FIGs. 7a to 11 are particularly advantageous to emergency situations because of the mobile nature of those implementations and the ability to quickly transport those implementations to areas where a stationary kiosk or booth may not be available or practical.

Medical implements such as a stethoscope 14, a blood pressure cuff 16, otoscope, ophthalmoscope, tuning fork, x-ray machine, MRI machine and the like may be attached to the kiosk 2. These medical implements allow the customer to conduct a self examination with the readings from the implements being electronically recorded and sent to a medical professional for evaluation. Medical implements that can be attached to and read by a computer system are available through AMD of Sunnyvale, California.

The kiosk 2 may also have a credit card reader 24 and a cash acceptor 22 in order to allow the customer to pay for medical services at the time they are rendered through the kiosk. A printer 8 is attached to the kiosk 2 so that the requested medical documents may be printed instantly on the appropriate paper. A portable video camera 12 may also be attached so that the customer may focus in on a rash, emergency condition, or other ailment that the medical professional would need to view.

The kiosk 2 also includes a power supply (not shown) to supply power to the kiosk 2, and a network connection (also not shown) to enable the transfer of data to and from the kiosk 2. Power supplies may include for example, a plug plugged into a standard outlet, or may include a battery or other power supply which does not require external connection. Similarly, the network connection to the kiosk 2 may include a direct connection to a data source, such as through a co-axial, telephone, network or other cable connection, or may include, without limitation, a wireless connection, such as Bluetooth transmissions, satellite transmissions, WiFi or WiMax transmissions, cellular transmissions, radio or other frequency transmissions, and the like. Power and network connection possibilities and configurations are well known in the art and those of ordinary skill in the art will readily be able to properly configure implementations for power and network connection from the description provided herein.

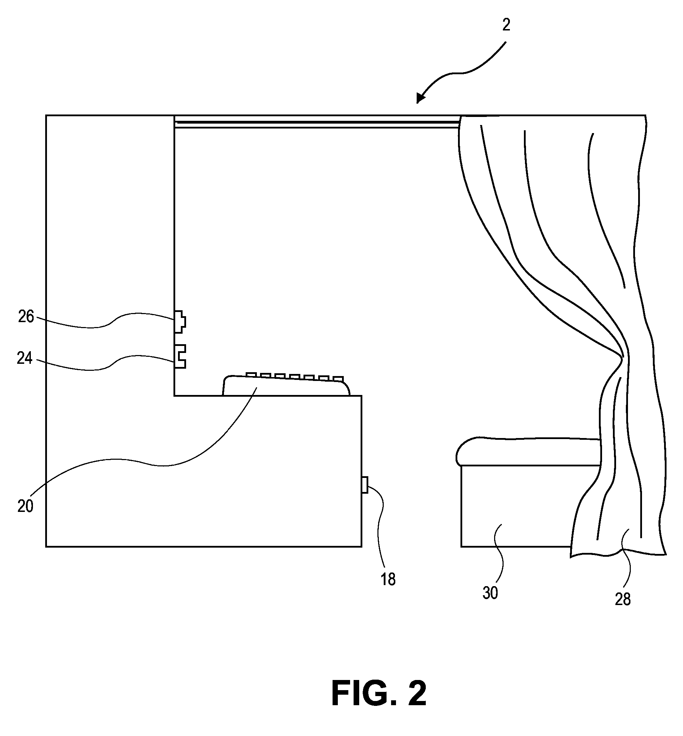

FIG. 2 illustrates the medical examination kiosk of FIG. 1 configured as a booth to provide privacy for customers receiving medical examinations. The kiosk 2 contains a seat 30 and a curtain 28 that may be closed to provide privacy.

FIG. 5 is a block diagram of a simplified example of the connectivity of an implementation of a medical services and prescription distribution system. The implementation shown in FIG. 5 includes a medical kiosk 138, a medical professional terminal 134, a medical information database 136 and an internet connection 132, each coupled to a system processor 130. Each of these connections may be accomplished by any combination of wired or wireless connection. Each or any of the components 138, 134, 136, 132 and 130 of the system may be located in a pharmacy, or in other remote location such as a doctor's home or office, particularly in cases where the kiosk 138 allows access to experts in certain medical fields, or even in a retail store, such as a grocery store, shopping mall, or any other place where it is desirable to have customers come. Those of ordinary skill in the art will understand how to build an appropriate configuration of components for a given situation from the disclosure provided herein.

Customer requests for medical documents and medical examinations made through the kiosk 138 are transmitted electronically to and processed by the system processor 130. Information contained within the document request is compared with previous medical data within the medical information database 136, and is approved by a doctor reviewing the circumstances of each request and each medical examination through the medical professional terminal 134. Return customers may be given a user ID and password to avoid the need to reenter basic personal information. It should be understood that any or all of the components of the system may be implemented using conventional personal computers associated as a network, provided the medical kiosk 138 includes a camera associated therewith and programmed to take a facial image of the user proximate the time of the examination.

The medical kiosk 138 may also include a video camera and voice interaction device to allow the customer and the medical professional to interact. It is possible, however, that a customer could conduct a medical self examination, following instructions provided through the kiosk 138, and that the doctor could review the information received at a later time. In most implementations, however, the kiosk 138 will be configured for general public use as a kiosk, and the medical professional terminal 134 will more likely be configured as a personal computer. The doctor may approve the request and issue the medical document which is sent to a pharmacy or other entity if requested by the customer, or the document may simply be issued and printed at the kiosk 138.

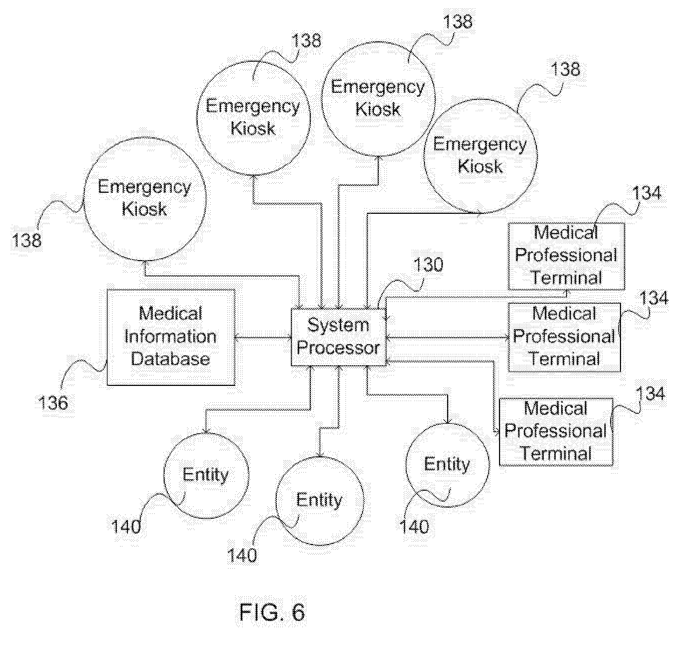

FIG. 6 is a block diagram illustrating a more complex example of the connectivity of a medical services or document distribution system. In this implementation, the system comprises a plurality of medical kiosks 138 that may be located at one or more physical locations, such as at a plurality of remote pharmacies, or two or more may be located in close proximity such as within a shopping mall where many people may desire to use the kiosks 138 simultaneously. Similarly, the system of FIG. 6 comprises a plurality of medical professional terminals 134 through which a plurality of doctors or medical professionals may issue and approve medical documents or conduct medical examinations. A plurality of entities 140 may be associated with the system so that customers may select the pharmacy or other entity to which the medical document will be sent. To achieve benefit from embodiments of the present invention, a pharmacy or other entity 140 need only have a fax machine or email to receive a medical document. The processor 130 may be configured to electronically fax a medical document to any entity 134. However, in more advanced systems, the entity 134 may include a computer for receiving and reviewing the medical document electronically, and/or a printer, copier or fax machine for creating a hard copy of the medical document depending upon the needs of the entity. The entities 140 of the various implementations may be pharmacies, doctor's offices, hospitals, employers, schools, government offices, emergency response or treatment groups or any other entity to which the customer may need to send medical documents.

The entities 140, particularly pharmacies, may be adapted to send requested pharmaceuticals by carrier, such as is currently done with cyber-pharmacies but with the added security of comparing the customer's requests with the medical information database 136 (including the shipping address and the customer's picture). Software, such as that distributed by IQ Biometrix, Inc. of Fremont, California, exists to compare facial images and determine identity like a finger print. Use of this software to compare faces of each requestor of medical documents may help to eliminate fraud and situations where a customer requests multiple prescriptions using different names, and even different mailing addresses. Nevertheless, a visual comparison may also be sufficient to verify the requestor.

Additionally, the customer's picture may be included on a shipping label if prescription drugs are sent through the mail. This allows the delivery person to ensure the drugs were delivered to the correct person. Conventional cyber-pharmacy systems require a signature by the requestor when the delivery is made. This, however, does not ensure security as the signator may be signing a different name. Use of this system, for example, would enable customers to have their prescriptions forwarded to and filled by a Canadian or other out-of-country pharmacy for shipment to the customers' homes in the United States.

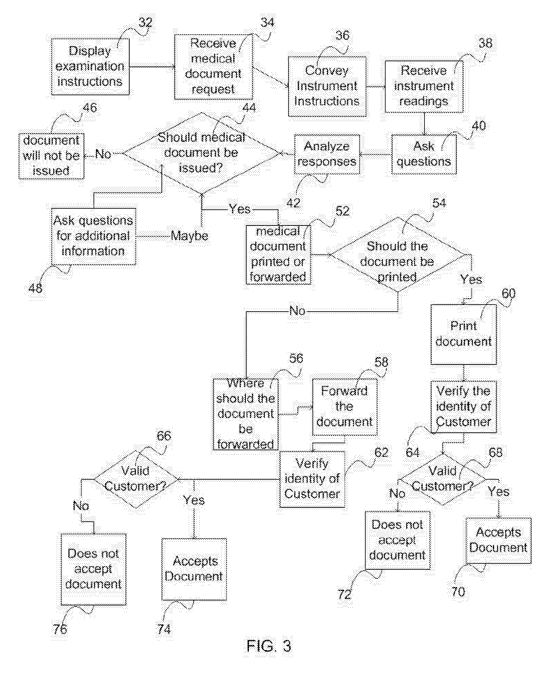

FIG. 3 includes a flow chart of a method of distributing medical documents and medical services illustrating one example implementation. The method of the implementation in FIG. 3 may be accomplished through a system having components such as those shown and described with reference to FIGs. 1-2 and 5-9. Instructions on how to begin a medical examination through the kiosk may be displayed on the kiosk or otherwise be provided through the kiosk (Step 32). Thereafter, the customer initiates, and the system receives, an examination and medical document request (Step 34). At the time the customer makes the request for a medical document, a camera associated with the system will capture an image of the customer. Part of making the request for a medical examination and medical documents for particular embodiments of the invention involves sliding a credit card through a credit card reader and approving the charge to the credit card or putting cash in the cash acceptor in order to pay for the services and documents to be provided. As an additional security measure, the customer's credit card billing address may be compared against the customer's indicated home address, or a possible shipping address in certain implementations, as a form of confirmation as to the identity of the customer.

The customer is then presented instructions on operating the medical implements attached to the kiosk (Step 36). The customer may operate the medical implements under the supervision of a medical professional through the interactive kiosk, or a medical professional, such as a nurse, may be present at the kiosk to assist or perform the examination for a doctor. The implement readings are then sent to the medical professional terminal (Step 38). The customer is presented with a questionnaire as part of the requesting process during which the customer is asked a series of questions necessary for the particular medical document requested (Step 40). There are typically a set of questions general to most document requests, i.e. customer's date of birth, height, weight, known allergies, current medications, known medical problems, alcohol and cigarette intake, past surgeries, and the like. Additionally, there may be a set of specific screening questions specifically directed to the specific medical document being requested. For example, if a prescription is requested or needed, specific questions may be directed to confirming that a particular medication is right for the customer. For example, and without limitation, with the genital warts drug Aldara®, the questions may include:
1) Do you have lesions that appear to be warts on or near your genital area?
2) Does your rash look more like that shown in Picture 1,2 or 3? (Three pictures are shown)
3) Have you ever been diagnosed with Genital Warts, Human Papilloma Virus (HPV) or Venereal Warts?
   A) If so, did you ever receive treatment for these warts?
   B) If so, which of the following methods were used when you received treatment? (A list of possible treatments are shown)
   C) Did you have success with any of those treatments?
   D) Did you have any adverse reaction or reaction that prompted your doctor to discontinue treatment or advise you to not use that treatment in the future?
4) Have you had an HIV test?
5) Which of the following have you been tested for? (A list of other sexually transmitted diseases is shown)
6) Do you use any steroids?
7) Have you had an organ transplant?

Additional validation information may be requested to ensure that the customer is legally eligible to receive the requested medication. For example, and without limitation, the validations may include, with a Yes or No response required for each one:
1) If you receive a prescription for medication from this medical examination, you will use the medication solely for your own personal therapeutic and medical needs, and not to distribute any of the medication to others.
2) You will promptly contact a physician for any necessary medical intervention should a complication or concern result related to the medical services and documents requested.
3) Should you be issued a prescription pursuant to the medical examination, you realize that there are risks as well as benefits to any medication, even over-the-counter drugs. You have been informed of the possible effects, risks and benefits of this medication.
4) You certify that you are 21 years of age or older.
5) You are permitted by law to receive these products or services in this region/country/locale.
6) You understand the side-effect of any medications prescribed or distributed at the examination.
7) You understand that you cannot have a prescription for a medication from more than one physician.
8) You certify that you are allowed by law to use the credit card you have selected below.
9) You understand that your credit card will be billed for this consultation and that if you choose to have medication dispensed from a licensed pharmacist, this too will be billed to your account if your request is approved.
10) You certify that you have, and will, answer all questions truthfully.
11) You understand that in accordance with federal laws and rules, your personal medical and other information will be kept in confidence and will not be used for any commercial uses.

Additional information questions regarding the identity of the customer may also be asked such as billing information, address, gender, etc., or for previous users of the system this information may be loaded from an associated database. The computerized medical questionnaire may be interactive, in that some answers from the consumer may determine which question will be asked next. The questions may also be formulated by a medical professional conducting the medical examination.

The responses to the questions are analyzed (Step 42) as the questions are answered or when the answers are submitted. This is particularly the case when a medical professional is conducting the medical examination. The questions may also be analyzed by a medical professional at some later time. The medical professional determines whether or not the medical document should be issued (Step 44). The customer should be informed if the medical document will not issue (Step 46). Otherwise, if it is determined that the medical document should be issued, the type of medical document that was requested comes into play. It is also possible that more information may be needed by the medical professional to issue the medical document (Step 48). If this is the case, more questions should be asked to clarify any concerns the medical professional may have.

If the medical professional approves the issuance of the medical document, the customer may be given the option to have the document printed from the kiosk and/or forwarded directly to some other entity (Step 52 and 54). If the customer wants the document printed from the kiosk, it can be printed immediately (Step 60). Though, if a medical professional is not present during the examination, the customer may need to return for the document or for a follow up. The medical document is printed with the customer's image attached in order to allow verification of the identity of the person to whom the medical document relates. When someone is presented with the medical document, that person may verify that the person presenting the document is the person to whom the document was issued (Step 64 and 68). Once the identity of the customer is verified, the document can be accepted (Step 70). Otherwise the document is not accepted (Step 72). Instead of, or in addition to a photograph of a customer's facial image, it is possible that other biometric comparisons may be made to verify the identity of the customer. Other biometric comparisons may include comparing fingerprints, DNA, retinal scans, or any other unique identifier.

If the customer prefers to have the medical document forwarded to another entity, information is gathered about that entity (Step 56), such as where the entity is located, an email address or fax number, and the like. The document is then forwarded with the customer's image attached (Step 58). This allows the entity to which the document is presented, to verify that the customer is the person to whom the document was issued (Steps 62 and 66). If the identity of the customer is verified, the entity may accept the document otherwise, the entity can reject it (Steps 74 and 76).

Information gathered by the kiosk is stored in a medical database for further reference. The customer's request establishes a specific record of who requested the medical document and who was examined. It provides not only a confirmation that the customer was asked all of the appropriate questions and answered them appropriately, but also that a specific photo record was made of who requested the document, answered the questions and participated in the examination. This record is one that is generally not created in a conventional doctor's office when a medical document is requested.

An initial determination may be made from the response received from the medical database as to whether the customer should receive the medical document and pass that along with the customer's responses. For example, and without limitation, if the customer has frequently requested the same prescription medication without just cause, the same facial image is associated with more than one name, or more than one facial image is associated with the same name, software associated with the system may identify the inconsistency. This may be indicated to the doctor along with the medical document request. Also, if the database is aware of a conflicting prescription previously received by the customer that is not identified in the customer's responses, if previous responses are inconsistent with current responses, or if the indications otherwise indicate that the customer should not be using the requested medication, this too may be identified and indicated to the doctor along with the medical document request.

As will be clear to those of ordinary skill in the art, the medical information database used in particular implementations included in this description may be configured in many different ways and may include any number of different types of information. Those of ordinary skill in the art will be readily able to design and build an appropriate database depending upon the needs of a particular system and the information desired to be stored and retrieved by system administrators. In particular implementations, each request for a medical document along with all responses and all relationships between requests may be tracked, queried and searched. Such a system would increase the usefulness of the database in providing security against fraudulent prescription requests is dependent, in part, upon the ability to search and compare existing records.

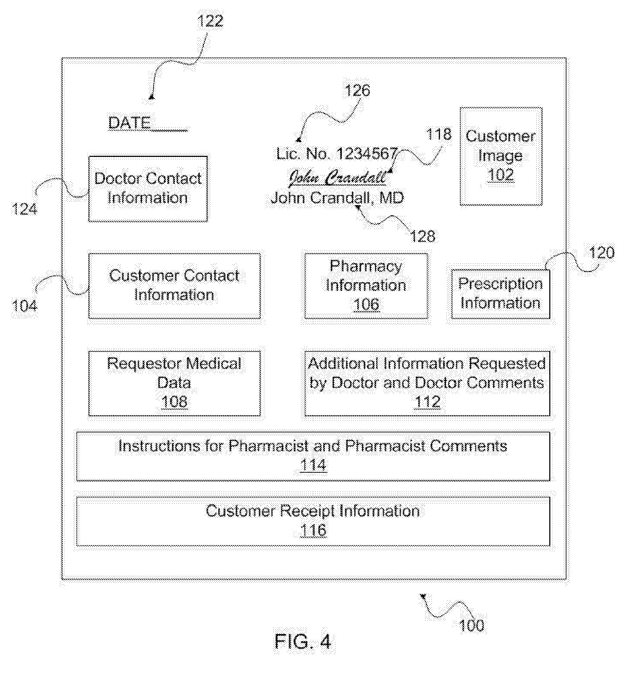

FIG. 4 illustrates an example of a prescription 100 generated by a particular implementation of a medical document system. A prescription 100 may include any or all of the elements described with reference to FIG. 4, in whatever combination is necessary or desired for a particular system. The prescription 100 shown and described with regard to this particular implementation is intended as a non-limiting example. A prescription 100, or conditional prescription, includes an image of the customer 102, the customer's contact information 104, information regarding the pharmacy 106 to which the customer indicated the prescription should be sent, and pertinent medical data 108 relating to the customer. The prescription 100 also includes a section for comments from the doctor 112, a section for specific instructions to the pharmacist and comments by the pharmacist 114, and a section for payment and perhaps delivery receipt information for the customer 116. Like a conventional prescription, the prescription 100 may also include a doctor's signature 118, prescription information 120 regarding the medication, dosage information, refills prescribed, and a date 122 for the prescription. Particular implementations of the prescription may also include the doctor's full contact information 124, license number 126, and title below the signature 128 for clarity. Other medical documents may have similar fields for follow-up questions and other information.

FIGs. 7a to 7c illustrate views of an implementation of a mobile emergency medical kit 150. This implementation includes a carrier 152 in which various medical implements may be stored. Medical implements are implements that may be used to assess information about a physiological or other medical condition of a patient. There are many various medical implements known and used. Many are already disclosed in this disclosure. The medical implements shown in FIG. 7a are electronic medical implements configured to receive stimuli and convert the stimuli into electronic data for transmission to an electronic data collector 154. Examples of electronic medical implements included in FIG. 7a are a stethoscope, 158, a blood pressure gauge 160, a pen light or video or still camera 162, and a blood composition monitor 164, such as an oxygen level sensor or a blood sugar (glucose) level sensor.

Another type of implement that may be included is an identification card reader 166 configured to scan or otherwise read information from an identification card such as a credit card, driver's license or other identifying card. Other medical implements, such as a thermometer, an ultrasound generator, and any other medical implementation commonly used in the industry for gathering physiological data may also be included.

With the recent improvements in electronics programming, the reduction in the size of electronics, and the ability of electronics to perform multiple differing functions and interface with different equipment, the medical implements may be programmed to gather and transmit the data to the electronic data collector 154 for analysis, some of the data may be analyzed by the electronic medical implement, and/or some of the data may be transmitted to the emergency response system for final analysis. Those of ordinary skill in the art are readily aware of electronic versions of common medical implements, how to adapt those electronic medical implements to associate with electronic data collectors, and how to best interpret and analyze the data received from the implements.

The implementation of FIG. 7a includes a fold-over flap 168 to help in retaining the implements in the carrier 152 when the carrier is rolled or folded shut. The handle 170 of this particular implementation is adapted to include hook and loop fasteners (such as Velcro®) along a section 172 of the handle to coordinate with corresponding components on the carrier 152 when the carrier 152 is rolled. FIG. 7b illustrates a side view and FIG. 7c illustrates an end view of the mobile emergency medical kit 150 implementation rolled with the handle fastened.

Other types and styles of carriers, including boxes and molded trays are known in the art and the specific style of carrier is not crucial to this invention. The carrier is provided as a convenience for packaging and/or maintaining the assembled unit as a kit. It is contemplated that each of the medical implements may be individually wrapped and sealed in sterile packaging typical of medical implements prior to use. Such medical implements may be created as disposable, recyclable, or reusable implements as is also common in the art of medical implements. As will be clear to those of ordinary skill in the art, the kit may include other electronic and non-electronic medical implements as well. For example, a conventional first aid kit may be included in the mobile emergency medical kit. Additionally, more complex medical devices such as, and without limitation, a heart defibrillator may be included.

FIG. 8 illustrates an implementation of an electronic data collector 154. The electronic data collector 154 includes a display 174, a plurality of function buttons 176, and an emergency button 178. The emergency "E" button may be configured to indicate a medical emergency so that connection to a medical professional will be accelerated. Data ports 180, such as USB ports or other data connector ports 182 may be used to allow connection between medical implements, such as the pen camera 162 shown, and the electronic data collector 154. One benefit of USB ports is that power may be supplied to the medical implement through the USB port instead of requiring the medical implement to provide its own power supply. To interface with the electronic data collector 154, a USB connector 182 or other suitable cable connector may be used.

In lieu of wired data communication between the medical implements and the electronic data collector 154, wireless communication may be used. Such wireless communication devices include, but are not limited to, Bluetooth, radio frequency, WiFi, WiMAX, satellite or cellular signals, or other wireless frequency signals known in the art. Because it is anticipated that the medical implements and the electronic data collector 154 will adequately function on simple communication schemes and are not required to include complex data transfer procedures. For the exemplary purposes of the Figures, the communications used by the kit are described as being incorporated into the electronic data collector 154. As will be understood by those in the electronic arts, however, the communications electronics may be fully incorporated within the electronics for the electronic data collector, or may simply be associated with the electronic data collector. When it is stated herein that communication is had with the electronic data collector, it is intended to mean that electronic communication is had either directly with the electronic data collector or indirectly with the electronic data collector through an associated component.

Power may be provided to the electronic data collector 154 through any number of known power sources such as rechargeable or non-rechargeable batteries, an electrical outlet, an automobile power connector such as a cigarette lighter, and the like. Because particular implementations of aspects relating to mobile emergency medical kits involve the kits being stored in an automobile or an airplane, power supplies conventionally located on such vehicles are a logical choice for adaptation. Similar to smoke detectors used to protect homes from fire, the electronic data collector 154 may be adapted to include a warning light or sound that activates when the battery is running low.

Though it cannot be shown on the drawing of FIG. 8, the electronic data collector 154 transmits, with data it gathers from a medical implement attached thereto, a unique identifier to an emergency response system. This allows the emergency response system to identify the kit being used, possibly identify who owns it, and determine whether there are electronic methods of identifying the location of the kit being used, such as through the On-Star® System or Lo-Jack® System installed on the vehicle or perhaps installed as part of the electronic data collector 154 or elsewhere in the kit.

One particular advantage of an aspect of the mobile emergency medical kit's electronic data collector that was previously unavailable to the art is the ability to wirelessly and transmit significant amounts of data over long distances in the short amount of time needed for an emergency situation. It is because of this improvement that a wide scale mobile emergency medical system can now be implemented so effectively. One such new wireless technology that is currently being implemented in many computer systems is the WiMAX technology, based upon IEEE standard 802.16. Prior to the most recent implementations, significant wireless data transmission over long distances made wireless implementation of emergency medical data impractical because data speeds were too slow to transmit the necessary medical data and long distance data transmission was not reasonably possible. WiMAX, in particular, enables transmission frequencies of approximately 2.5 GHz to 5 GHz for 50 kilometers or more. Prior high speed data transmission was limited to much smaller distances and lower speeds. New wireless technologies now enable practical applications of long distance mobile emergency medical data transmissions.

FIG. 9 illustrates a mobile emergency medical kit incorporated into a vehicle dash board 184. The electronic data collection portion of the kit is mounted in the dash board 184 and includes a display 186, one or more function buttons 188, an emergency button 190, and one or more data ports 194. A medical implement carrier including medical implements may be stored in the glove compartment 192, under a car seat, or at some other location in the vehicle.

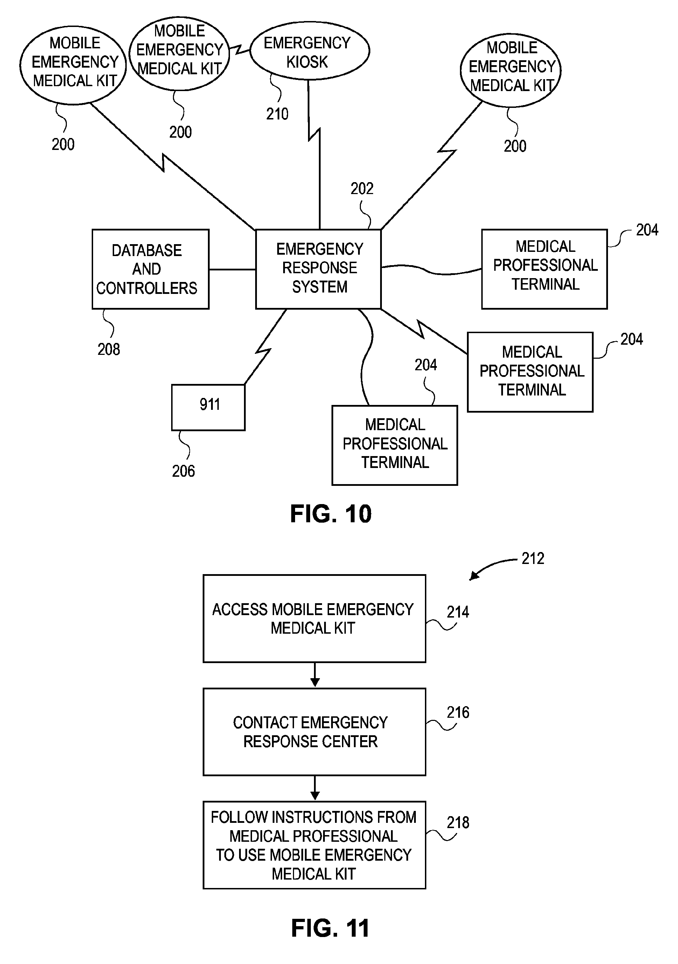

FIG. 10 illustrates an implementation of a Mobile Emergency Response System. A plurality of mobile emergency medical kits 200, for example the mobile emergency medical kit implementations described with reference to FIGs. 7 to 9, is each configured for wireless communication with a central Emergency Response System 202. Distinct from a conventional call center, the Emergency Response System 202 is configured to receive data indicative of physiological conditions of patients through the medical implements associated with each of the mobile emergency medical kits 200. The Emergency Response System 202 is further associated with a plurality of Medical Professional Terminals 204 that are locally or remotely coupled to the Emergency Response System 202. As illustrated by FIG. 10, a 911 operator terminal 206 may be one of the medical professional terminals coupled to the Emergency Response System 202 in the case that a medical professional cannot provide an adequate response. A customer, medical information, informational, or other database and whatever additional controllers are needed 208 may also be coupled to the Emergency Response System 202.

Incoming communications are routed to the appropriate Medical Professional Terminal for best assistance to the incoming communication, much like a 911 call center. One significant advantage of a mobile emergency medical kit 200 is that it has the additional benefit of providing laypersons with medical implements that may be used to assist the medical professional by providing physiological data not previously available to medical professionals during a remote diagnosis for a medical emergency. A medical professional associated with the Medical Professional Terminal 204 can further instruct the user communicating through the electronic data collector to use the medical implements appropriately to collect relevant data to answer the medical professional's questions. Alternatively, instruction in use of the medical implements may be provided to the customer at the time the medical kit is provided to the customer.

As will be evident to those of ordinary skill in the art, mobile emergency medical kits and associated emergency response systems configured to receive data from remote electronic medical implements are useful and advantageous in all locations where a large number of people exist or emergencies may occur. For example, and without any limitations, in an airplane, in a vehicle, on a construction site, at home, in public buildings such as retail stores, amusement parks and airports, and the like. By providing a medical emergency response network that not only assists in the medical emergency through analyzing an observer's verbal explanation of the emergency, but also collects physiological data relating to the patient, more accurate medical information can be collected more quickly, and better medical procedures can be provided more quickly.

FIG. 11 illustrates an implementation of a method of using a mobile emergency medical kit 212. A person who is experiencing or witnessing a medical emergency ("user") first accesses a mobile emergency medical kit (Step 214). For persons at home, the kit may be stored in the kitchen or other convenient location. For a vehicle, the kit may be in the glove compartment or other storage compartment or under a seat of the vehicle. For some vehicles using On-Star® or a similar system, the electronic data collector may be incorporated into or placed adjacent to the On-Star® system on the console or dash board of the vehicle. Because the electronic data collector is configured for wireless communication to a remote location, it is particularly advantageous for use in a variety of locations with the same kit; thus enabling endless adaptation to various use environments and locations with the same kit equipment.

After accessing the kit, the user contacts the Emergency Response Center (Step 216) through the wireless communications portion of the electronic data collector. Contacting the Emergency Response Center may simply involve pressing an emergency button on the electronic data collector. Alternatively, it may involve pressing a "non-emergency" button on the device or even pressing a sequence of buttons to complete the contact connection. Once the Emergency Response Center is contacted, the user can inform the medical professional of the medical emergency, follow instructions from the medical professional on how to use the medical implements associated with the mobile emergency medical kit to get the physiological data needed (if any), and collect the needed data using the kit (Step 218). The Emergency Response Center's customer database may include a call history and even a medical history associated with the unique identifier associated with the call to ensure the customer's previous needs that may be relevant to the present call are known to the medical professional. Using the medical implement may involve associating a medical implement with the electronic data collector by plugging it into the device or turning it on.

Once the physiological data has been collected by the user and transmitted to the medical professional for review, the medical professional can provide more meaningful assistance to the user for assisting in the medical emergency. The physiological data may then be transmitted by the emergency response system or by the mobile emergency medical kit to the emergency medical personnel who arrive at the scene to assist in their diagnosis and treatment, and/or transmitted to the hospital to which the patient is transferred if desired.

## Claims

1. A medical service distribution system comprising:
a customer interface with a computer system; and
a camera associated with the customer interface and configured to capture an image of a customer at the customer interface;
wherein the customer interface is configured to receive a request for a medical document from the customer through an interface input, capture a facial image of the customer requesting the medical document proximate a time of the request through the camera, and generate, in response to the request, the requested medical document including the facial image of the customer requesting the medical document.

2. The medical service distribution system of claim 1, wherein the medical document is a prescription for a prescription drug or a letter concerning the customer's health.

3. The medical service distribution system of claim 1, wherein the customer interface is adapted to allow the customer to see and speak to a medical professional.

4. The medical service distribution system of claim 1, further comprising one or more of the following:
a) medical instruments attached to the system in order to take medical readings from the customer,
b) a medical kit for emergency treatment,
c) a credit card or currency acceptor,
d) an emergency button, operable by the customer to summons professional medical help, e) at least one video camera, wherein the at least one video camera allows the customer to show a medical professional any physical symptoms that the customer may have,
f) at least one video camera, wherein the at least one video camera allows the customer to show a medical professional any physical symptoms that the customer may have and to allow the medical professional to monitor the customer's use of the customer interface.

5. The medical service distribution system of claim 1, wherein the medical document is printed directly from the system.

6. A method of providing medical documents, the method comprising:
receiving a request for a medical examination from a customer through a customer interface with a computer;
receiving information concerning the customer through the customer interface with a computer;
receiving a request from the customer for a medical document through the customer interface with a computer;
capturing a facial image of the customer requesting the medical document proximate a time of the request through the customer interface with a computer; and
generating a medical document with the customer's image in response to the request through the customer interface with a computer.

7. The method of claim 6, further comprising receiving responses from the customer to a plurality of medical questions related to the medical examination proximate the time of the request through the customer interface with a computer.

8. The method of claim 6, further comprising confirming an identity of the customer having the medical document by comparing the image on the medical document to the customer.

9. The method of claim 6, wherein the information received through the customer interface with a computer concerning the customer consists partially of instrument readings such as blood pressure, pulse rate and the like, and optionally the medical professional directs the customer in the use of medical instruments through the customer interface with a computer, wherein the medical instruments are part of the customer interface.

10. A method of issuing medical documents, the method comprising:
a medical professional communicating with a customer through a customer interface with a computer;
the medical professional directing the customer in taking readings using medical instruments, wherein the medical instruments are part of the customer interface with a computer;
the medical professional asking the customer medical questions through the customer interface with a computer;
the medical professional receiving a request for a medical document through the customer interface with a computer which captures a facial image of the customer requesting the medical document proximate a time of the request; and
the medical professional issuing, through the customer interface with a computer, the medical document attached to the facial image of the customer in order to allow verification of the identity of the customer as the owner of the medical document.
